# EUROPEAN PATENT APPLICATION

(11) **EP 4 108 222 A1**
(43) Date of publication of application: **28.12.2022**
(21) Application number: 21756888.0
(22) Date of filing: 15.02.2021
(51) Int. Cl.: A61J 3/00, B65B 1/30, B65B 57/10

(54) **DRUG IDENTIFICATION DEVICE AND DRUG IDENTIFICATION METHOD**

(30) Priority: 21.02.2020 JP 2020028454
(71) Applicant: FUJIFILM Toyama Chemical Co., Ltd., Chuo-ku Tokyo 104-0031 (JP)
(72) Inventor: OKUTSU, Hirokazu, Minamiashigara-shi, Kanagawa 250-0111 (JP); YOSHIDA, Tomohisa, Minamiashigara-shi, Kanagawa 250-0111 (JP)
(74) Representative: Jeffrey, Philip Michael
(86) International application number: PCT/JP2021/005416
(87) International publication number: WO 2021/166823

(57) **Abstract**

To provide a drug identification device and a drug identification method capable of eliminating an overlap of drugs in dose packaging bags. A drug identification device includes: an inlet configured to receive dose packaging bags in each of which drugs are packaged; a conveying path configured to convey the dose packaging bags received by the inlet; an imaging element configured to capture images of the drugs on the conveying path; a processor configured to identify the drugs based on the images of the drugs captured by the imaging element; a storing case arranged at an upstream side of the inlet and configured to store the dose packaging bags; and a vibration device configured to vibrate the storing case.

## Description

### BACKGROUND OF THE INVENTION

### 1. Field of the Invention

The present invention relates to a drug identification device and a drug identification method.

### 2. Description of the Related Art

In recent years, hospitals and pharmacies have introduced a dose packaging device and an audit device. The dose packaging device performs a dose packaging work for packaging drugs into dose packaging bags based on prescription data. The audit device performs an audit work for inspecting whether drugs are packaged into dose packaging bags discharged from the dose packaging device as per the prescription data.

In a dose packaging bag discharged from the dose packaging device, some drugs may overlap with each other. In a case where the drugs overlap with each other, the audit device cannot correctly perform the audit work in some cases. To solve such a problem, Japanese Patent No. 5886209 (hereinafter referred to as "Patent Literature 1") discloses, for example, a technique which uses vibrating means provided on an upstream side of a conveying path and above the conveying path. A dose packaging paper is shaken up and down in a vibration space provided between the vibrating means and the conveying path so as to properly separate a plurality of tablets from each other.

### Citation List

Patent Literature 1: Japanese Patent No. 5886209

### SUMMARY OF THE INVENTION

However, in Patent Literature 1, in the vibration space on a downstream side of the vibrating means, drugs inside the dose packaging bags are on a front side in an advance direction. Therefore, it is difficult to separate drugs even with vibration applied thereto, since the drugs overlap in the dose packaging bags. In addition, at the upstream side of the vibrating means, the dose packaging bags are supplied from the lower side to the vibrating means on the upper side, whereas at the downstream side of the vibrating means, the dose packaging bags are supplied from the upper side to the conveying path on the lower side. Therefore, tension of the dose packaging bags acts on the drugs inside the dose packaging bags, which makes it difficult to move the drugs.

The present invention has been made in view of such circumstances, and an object of the present invention is to provide a drug identification device and a drug identification method capable of eliminating an overlap of drugs in dose packaging bags.

A drug identification device according to a first aspect includes an inlet configured to receive dose packaging bags in each of which drugs are packaged, a conveying path configured to convey the dose packaging bags received by the inlet, an imaging element configured to capture images of the drugs on the conveying path, a processor configured to identify the drugs based on the images of the drugs captured by the imaging element, a storing case arranged at an upstream side of the inlet and configured to store the dose packaging bags, and a vibration device configured to vibrate the storing case. According to the first aspect, it is possible to eliminate an overlap of the drugs inside the dose packaging bags.

In the drug identification device according to a second aspect, the vibration device vibrates the storing case in a horizontal direction. According to the second aspect, it is possible to vibrate the dose packaging bags in the horizontal direction so as to disperse the drugs in the horizontal direction inside the dose packaging bags.

In the drug identification device according to a third aspect, the vibration device vibrates the storing case in a direction perpendicular to a conveying direction of the dose packaging bags. According to the third aspect, it is possible to vibrate the dose packaging bags in a width direction so as to disperse the drugs in a width direction inside the dose packaging bags.

In the drug identification device according to a fourth aspect, the vibration device includes a motor and a cam attached to the motor. According to the fourth aspect, the storing case can be easily vibrated.

In the drug identification device according to a fifth aspect, the storing case includes a horizontal guide path aligned with a position of the inlet. According to the fifth aspect, the horizontal conveying path to the inlet may be extended, which makes it easier to disperse drugs in the conveying direction.

The drug identification device according to a sixth aspect, further includes a dispersion device configured to disperse the drugs inside each of the dose packaging bags, on the conveying path. According to the sixth aspect, it is possible to disperse drugs on the conveying path.

A drug identification method in a seventh aspect includes: a conveying step of conveying on a conveying path, dose packaging bags which are put into an inlet and in each of which drugs are packaged; an imaging step of capturing images of the drugs on the conveying path; and an identifying step of identifying the drugs based on the captured images of the drugs, and the drug identification method further includes a vibrating step of storing the dose packaging bags in a storing case arranged at an upstream side of the inlet and vibrating the storing case. According to the seventh aspect, it is possible to eliminate an overlap of the drugs inside the dose packaging bags.

In the drug identification method in an eighth aspect, in the vibrating step, the storing case is vibrated in a horizontal direction. According to the eighth aspect, it is possible to vibrate the dose packaging bags in the horizontal direction so as to disperse the drugs in the horizontal direction inside the dose packaging bags.

In the drug identification method in a ninth aspect, in the vibrating step, the storing case is vibrated in a direction perpendicular to a conveying direction of the dose packaging bags. According to the ninth aspect, it is possible to vibrate the dose packaging bags in a width direction so as to disperse the drugs in the width direction inside the dose packaging bags.

In the drug identification method in a tenth aspect, in the vibrating step, the storing case is vibrated before the dose packaging bags are put into the inlet.

In the drug identification method in an eleventh aspect, in the vibrating step, the storing case is vibrated after the dose packaging bags are put into the inlet. The tenth and eleventh aspects define preferable aspects of cases when the storing case is vibrated.

The present invention can eliminate an overlap of the drugs inside the dose packaging bags.

### BRIEF DESCRIPTION OF THE DRAWINGS

Figure 1 is a schematic diagram showing the configuration of a drug audit support system according to an embodiment.
Figure 2 is a block diagram showing the configuration of a receipt computer.
Figure 3 is an enlarged view of a packaging mechanism included in a dose packaging machine.
Figure 4 is a partially enlarged view of a drug identification device.
Figures 5A and 5B are partially enlarged views including a vibration device.
Figure 6 is a schematic configuration diagram of the drug identification device.
Figure 7 shows dose packaging bags in the state of being discharged from an outlet of the dose packaging device.
Figure 8 shows states of drugs in a dose packaging bag before and after vibration.
Figure 9 shows a modified example of a storing case.

### DESCRIPTION OF THE PREFERRED EMBODIMENTS

Hereinafter, a preferred embodiment of the present invention is described based on the accompanying drawings. The present invention is described with the preferred embodiment described below. Changes are possible by many methods without departing from the scope of the present invention, and embodiments other than the present embodiment are also available. Therefore, all the changes within the scope of the present invention are embraced in the claims.

Here, components designated by the same reference numeral correspond to similar elements having similar functions. In this description, numerical ranges expressed with "-" includes an upper limit and a lower limit in the expression of "-".

Drug prescription works performed in hospitals, pharmacies, and so on, roughly include: a prescription data input work; a picking work; an automated dose packaging work; an audit work; and a drug administration guidance and prescription work. In the drug administration guidance and prescription work, a pharmacist performs an audit, and then provides a patient with drug administration guidance and prescription of drugs (dose packaged drugs) packaged to each packaging bag.

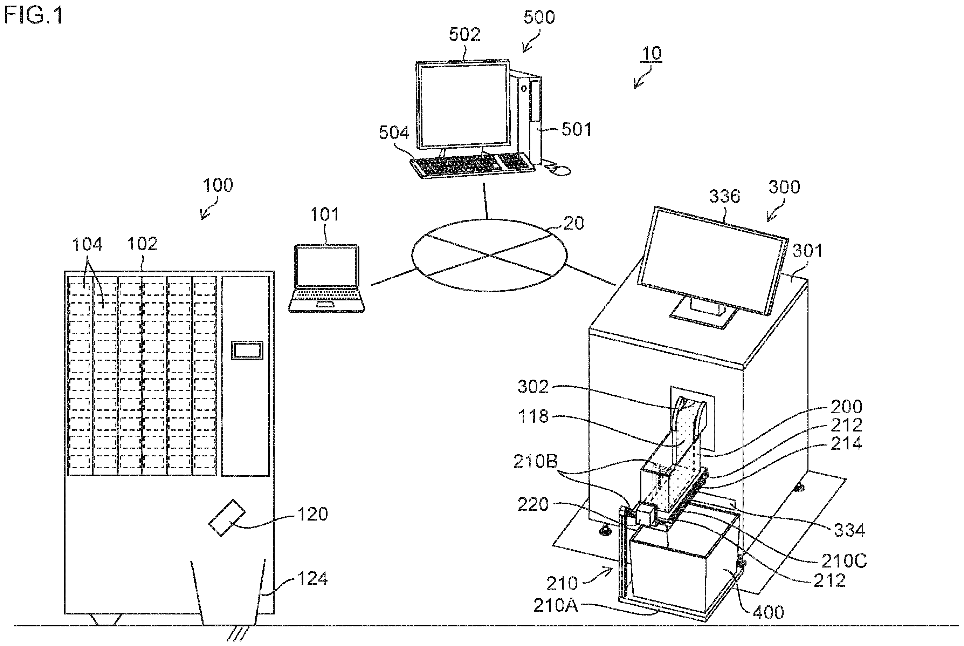

Figure 1 is a schematic configuration diagram showing a drug audit support system. As shown in Figure 1, a drug audit support system 10 includes a dose packaging device 100, a drug identification device 300, and a receipt computer 500.

In the prescription data input work, a pharmacist inputs prescription data written on a prescription into a receipt computer 500. Examples of the prescription data may include name and age of a patient, a drug type or a drug name, a drug quantity, drug usage, or a drug dosage. In the specification, the term of the drug type is synonymous with drug class or drug category.

The pharmacist then operates the receipt computer 500 to print the prescription data from a printer (not shown) connected to the receipt computer 500.

In the picking work, the pharmacist picks up drugs corresponding to the prescription data from pharmacy shelves, based on the prescription data on a print output from the printer. Examples of the drugs may include a tablet drug and a capsule drug. Here, in the picking work, an automated picking device that automatically picks up drugs based on the prescription data input into the receipt computer may be used, for example.

In the specification, the terms of "upper" and "lower" directions respectively correspond to "upper" and "lower" directions in a case where the drug audit support system is installed in normal use. As for the terms of "longitudinal" and "lateral", the term "longitudinal" corresponds to a "vertical (V)" direction, and the term "vertical" includes substantially vertical. For example, when it is assumed that the vertical direction is 0°, a range of ± 20° from 0° is included in "substantially vertical (or vertical)". The term "lateral" corresponds to a horizontal (H) direction, and the term "horizontal" includes substantially horizontal. For example, when it is assumed that the horizontal direction is 0°, a range of ± 20° from 0° is included "substantially horizontal (or horizontal)". As for the terms of "longitudinal attitude" and "lateral attitude", the "longitudinal" and "lateral" are determined based on a shorter direction (or widthwise direction) of continuous dose packaging bags. The terms of "upstream" and "downstream" relate to a conveying direction of the dose packaging paper or the dose packaging bags. The "downstream" corresponds to a side toward which the dose-packing paper is conveyed with respect to a certain reference, and the "upstream" corresponds to a side opposite to the side toward which the dose-packing paper is conveyed.

### <Receipt Computer>

The receipt computer 500 includes: a control device 501 having a processor; a display device 502 having a display device; and an input device 504 having a keyboard. The receipt computer 500 is connected to an in-hospital network 20, for example.

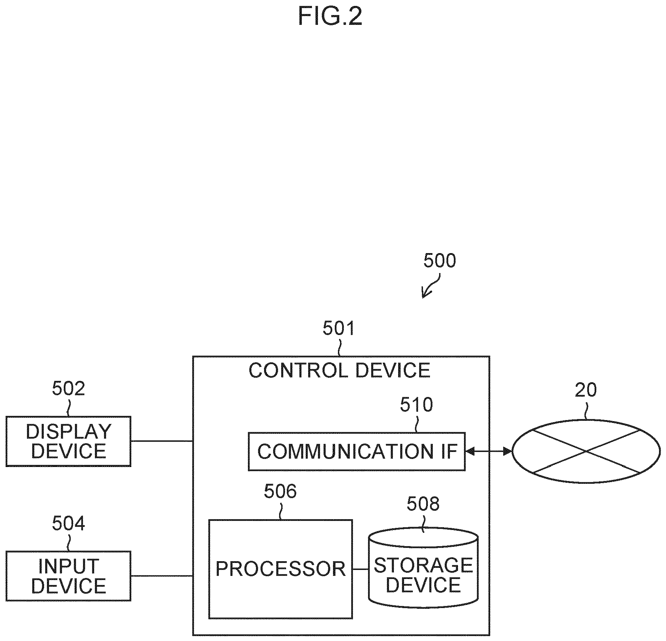

Figure 2 is a block diagram showing a configuration of the receipt computer 500. As shown in Figure 2, the control device 501 of the receipt computer 500 includes: a processor 506 that performs various control; a storage device 508 that stores various data; and a communication interface 510 that performs data communication with external networks. The control device 501 is electrically connected to the display device 502 and the input device 504. The control device 501 of the receipt computer 500 is connected to the in-hospital network 20 via the communication interface 510.

### <Dose Packaging Machine>

As shown in Figure 1, the dose packaging device 100 includes: a dose packaging machine controller 101 that is connected to the network 20; and a cabinet 102 that is controlled by the dose packaging machine controller 101 to perform the dose packaging work. The dose packaging device 100 includes a plurality of feeders 104 which respectively store a plurality of drugs. The plurality of feeders 104 may be arranged longitudinally and laterally. The plurality of feeders 104 may also include the feeders 104 arranged on the rear side as viewed from the front side. The feeders 104 can drop drugs stored therein, downward one at a time.

The dose packaging machine controller 101 includes a processor not shown. Based on the prescription data from the receipt computer 500, the processor can select required feeders 104 and causes the feeders 104 to drop drugs stored therein downward. The drugs for one package are dropped down. Each of the feeders 104 may include: a cassette which stores drugs; and a chute which guides the drugs down from the cassette.

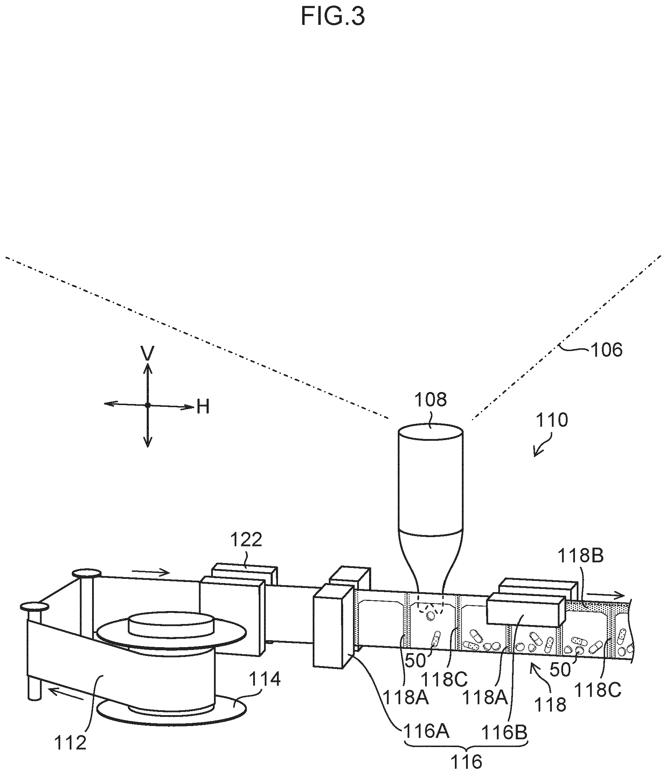

As shown in Figure 3, the dose packaging device 100 includes a hopper 106 on the lower side of the feeders 104. The hopper 106 is a cylindrical member with a wide opening on the upper side thereof and a narrow opening on the lower side thereof. The narrow opening is narrower than the wide opening. The hopper 106 collects drugs 50 dropped from the feeders 104 (see Figure 1) arranged above the hopper 106, and gathers the drugs to one spot on the lower side. On the lower side of the hopper 106, a charge pipe 108 is provided.

A packaging mechanism 110 is provided below the charge pipe 108. The drugs 50 collected with the hopper 106 are guided to the packaging mechanism 110 through the charge pipe 108. The charge pipe 108 is a cylindrical member extending in a vertical direction. The charge pipe 108 may be circular or elliptical in cross section. The charge pipe 108 may also be in a cylindrical or pyramid shape (truncated cone shape). The charge pipe 108 may be in any shape as long as the charge pipe 108 can guide the drugs 50 to the packaging mechanism 110. The drugs 50 are, for example, tablets, capsules, and etc.

The packaging mechanism 110 includes: a supply mechanism 114 which feeds the dose packaging paper 112; and a heat seal mechanism 116 which heat-fuses the dose packaging paper 112. The dose packaging paper 112 is made of a heat-fusible material. The dose packaging paper 112 is in a state where a long-length sheet is folded in two in a short-side (widthwise) direction and then wound into a roll shape.

For example, the heat seal mechanism 116 has: a longitudinal heat head 116A arranged longitudinally; and a lateral heat head 116B arranged laterally. The heat seal mechanism 116 can form longitudinal seal parts 118A and lateral seal parts 118B on the dose packaging paper 112 which is being conveyed.

The supply mechanism 114 includes: a shaft which holds the rolled dose packaging paper 112; and a drive motor which rotates the shaft, or the like, for example. The dose packaging machine controller 101 can rotationally drive the drive motor intermittently or continuously.

The dose packaging paper 112 is conveyed in a longitudinal attitude in which a double folded part is directed downward. For example, the longitudinal heat head 116A of the heat seal mechanism 116 forms the longitudinal seal parts 118A on the dose packaging paper 112. The longitudinal heat head 116A of the heat seal mechanism 116 is equipped with a perforation forming device (not shown). The perforation forming device includes, for example, a plurality of blades that can penetrate the dose packaging paper 112. In a case where the longitudinal heat head 116A heat-fuses the dose packaging paper 112 from both sides, the perforation forming device forms perforations 118C on the longitudinal seal part 118A. The dose packaging paper 112 becomes into a half-closed state.

Then, the half-closed dose packaging paper 112 passes through the charge pipe 108. The drugs 50 for one package are supplied from the charge pipe 108 to the half-closed dose packaging paper 112. Then, the lateral heat head 116B of the heat seal mechanism 116 forms a lateral seal part 118B.

The dose packaging paper 112 is partitioned by the heat seal parts (the longitudinal seal part 118A and the lateral seal part 118B) and the perforations 118C, so as to form individually partitioned dose packaging bags 118. Note that the continuous dose packaging bags 118 may be separated into individual dose packaging bags 118 by cutting the continuous dose packaging bags 118 along the perforations 118C.

The packaging mechanism 110 may include a printhead 122. The printhead 122 prints on the dose packaging paper 112. Information to be printed includes, for example, a patient name, a drug name, and its usage.

As shown in Figure 1, the dose packaging device 100 discharges the continuous dose packaging bags 118 from an outlet 120. The outlet 120 is a rectangular opening.

The outlet 120 is configured such that its longitudinal side is tilted by about 45° to the right with respect to the vertical direction in a front view. The dose packaging bags 118 discharged from the outlet 120 are stored in a storing case 124 arranged below the outlet 120.

### <Drug Identification Device>

As shown in Figure 1, the drug identification device 300 has a cabinet 301. The cabinet 301 includes: an inlet 302 which receives the continuous dose packaging bags 118; and an outlet 334 which discharges the continuous dose packaging bags 118. The drug identification device 300 according to the present aspect is applicable to drug audit and drug audit support, for example.

On the cabinet 301, a display device 336 is provided. The display device 336 displays various information. The various information includes, for example, prescription information for patients, dose packaging information, collation results, or other information input from the receipt computer 500 or the dose packaging machine controller 101.

The inlet 302 and the outlet 334 are arranged on an upper side and a lower side along the vertical direction. In the present embodiment, the inlet 302 is arranged on the upper side and the outlet 334 is arranged on the lower side. At the downstream side of the outlet 334, a storing box 400 is arranged. The storing box 400 stores the dose packaging bags 118 discharged from the outlet 334.

At the upstream side of the inlet 302, a storing case 200 which stores pre-audit dose packaging bags 118, a frame 210 which supports the storing case 200, and a vibration device 220 which vibrates the storing case 200 are provided. The frame 210 and the vibration device 220 can be separated from the cabinet 301. The storing case 200 may be the storing case 124 used in the dose packaging device 100, or may be a different case.

The frame 210 includes: a base 210A having four sides that are provided so as to surround the storing box 400; and two posts 210B extending vertically from the base 210A. The frame 210 includes: guide rails 212; and a base plate 214 attached to the guide rails 212.

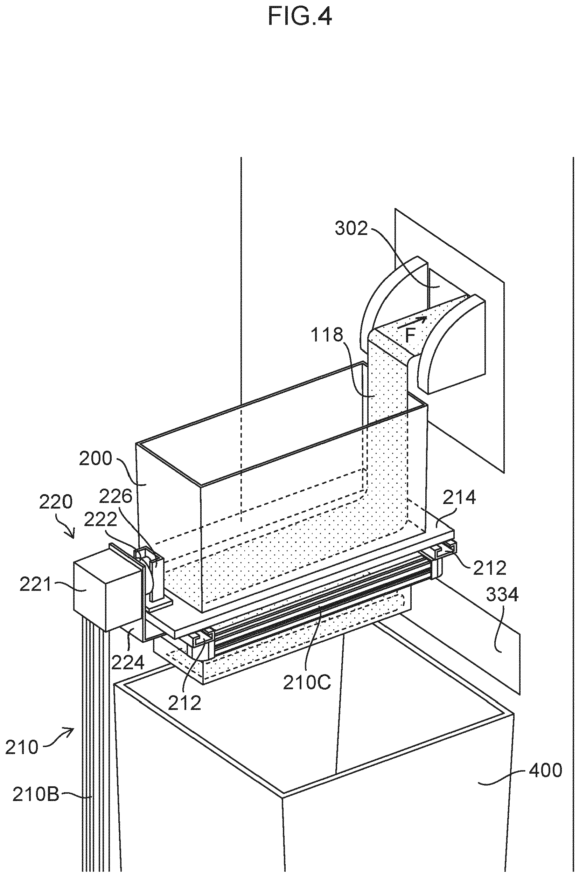

Figure 4 is an enlarged view of the drug identification device 300. As shown in Figure 4, the drug identification device 300 includes: an upper base 210C having two sides extending horizontally from distal ends of the respective two posts 210B; and one side which couples the two sides. The upper base 210C has a U-shape made of the three sides. The two sides which extend in the horizontal direction are perpendicular to a conveying direction F of the dose packaging bags 118.

Guide rails 212 are respectively provided on the two horizontally extending sides of the upper base 210C, in parallel to the two sides. The base plate 214 is slidably attached to the two guide rails 212.

An opening is formed on the base plate 214, and the storing case 200 is inserted to the opening. The storing case 200 has a wall surface with a step formed thereon. Since the step of the storing case 200 and a peripheral edge of the base plate 214 defining the opening are in contact with each other, the base plate 214 can support the storing case 200.

The base plate 214 can move along the guide rails 212 while supporting the storing case 200. The movement direction is a horizontal direction and perpendicular to the conveying direction of the dose packaging bags 118. The movement may be a reciprocating linear motion.

The vibration device 220 includes: a motor 221; and a cam 222 attached to the motor 221. The motor 221 is held on the upper base 210C of the frame 210 via a bracket 224. The cam 222 rotates as the motor 221 rotates.

The base plate 214 has a U-shaped coupling member 226 which extends upward in the vertical direction, with respect to the base plate 214. The cam 222 is housed in a space of the U-shaped coupling member 226.

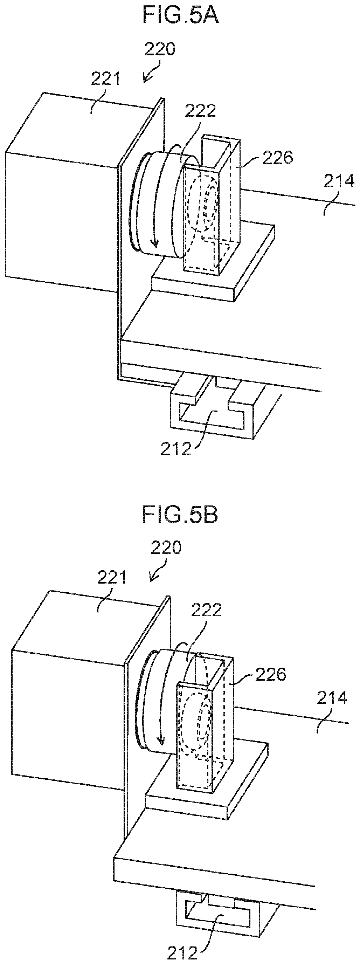

Figures 5A and 5B are partially enlarged views including the vibration device 220. The cam 222 is attached to the motor 221 as shown in Figures 5A and 5B. The cam 222 is eccentric to a shaft center of the motor 221. The cam 222 rotates as the motor 221 rotates. As the cam 222 rotates, the coupling member 226 moves back and forth. Since the coupling member 226 is coupled to the base plate 214, the base plate 214 moves back and forth along the guide rails 212. The storing case 200 inserted into the base plate 214 moves back and forth in synchronization with the movement of the base plate 214. The vibration device 220 having the motor 221 and the cam 222 can vibrate the storing case 200 (not shown) via the coupling member 226 and the base plate 214. The vibration can be controlled by a rotation speed of the motor 221, a size of the cam 222, etc.

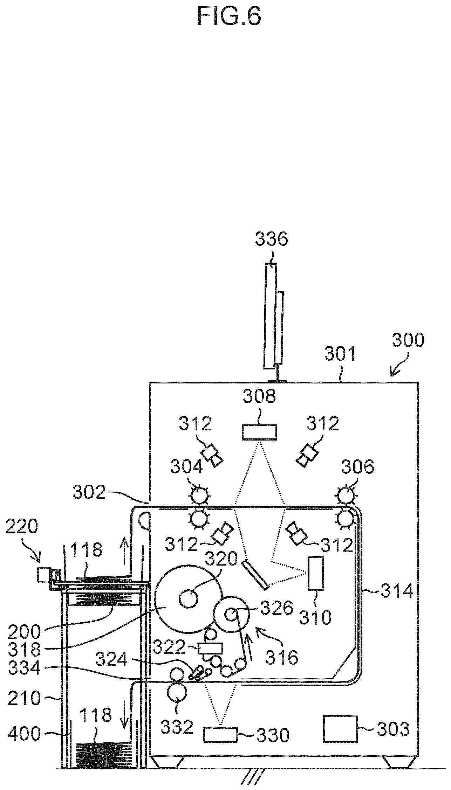

Figure 6 is a schematic configuration diagram of the drug identification device 300. As shown in Figure 6, at the upstream of the inlet 302, the drug identification device 300 includes: the storing case 200; the frame 210 which supports the storing case 200; and the vibration device 220 which vibrates the storing case 200 as described above.

The cabinet 301 includes a processor 303 which controls the entire drug identification device 300. The processor 303 of the drug identification device 300 is connected to the network 20 (see Figure 1) and prescription information may be obtained from the receipt computer 500 and the dose packaging machine controller 101.

A pair of first conveying rollers 304 and a pair of second conveying rollers 306 are provided at the downstream side of the inlet 302 inside the cabinet 301. The first conveying rollers 304 are upstream-side conveying rollers and the second conveying rollers 306 are downstream-side conveying rollers. The inlet 302 is oriented so that the dose packaging bags 118 may be put into the inlet 302 in a lateral attitude (in a horizontal state). The first conveying rollers 304 and the second conveying rollers 306 respectively hold (nip) the lateral seal parts 118B of the continuous dose packaging bags 118 from the upper and lower directions. Because the lateral seal parts 118B are held, it is possible to avoid the drugs 50 being trapped and damaged by the first conveying rollers 304 and the second conveying rollers 306. Here, in Figure 6, the drugs 50 are not shown.

On a conveying path between the first conveying rollers 304 and the second conveying rollers 306, an imaging area is provided. In the imaging area, a first camera 308 is arranged on the upper side of the conveying path, and a second camera 310 is arranged on the lower side of the conveying path. The first camera 308 and the second camera 310 are digital cameras, for example. Digital cameras include an imaging element having a charge coupled device (CCD), a complementary metal oxide semiconductor (CMOS), or the like.

A plurality of light sources 312 are respectively arranged on the upper side and the lower side of the conveying path. On the upper side of the conveying path, four light sources 312 are arranged at equal intervals on the same circumference around an imaging optical axis of the first camera 308. Similarly, on the lower side of the conveying path, four light sources 312 are arranged at equal intervals on the same circumference around an imaging optical axis of the second camera 310.

The imaging area of the conveying path is made of a transparent member. The first camera 308 and the second camera 310 image (image-capture) the drugs 50, which are dose packaged in the dose packaging bags 118, to be conveyed, from the upper and lower directions. In the imaging area, the dose packaging bags 118 are put in a horizontal state. A surface enclosed by the longitudinal seal part 118A, the lateral seal part 118B, and a double folded part of the dose packaging bag 118 is put in the horizontal state. It is preferable to provide a dispersion device, which is not shown, in the imaging area.

The first camera 308 and the second camera 310 capture images of the drugs 50, for each package. The processor 303 determines whether or not an overlap of the drugs 50 is present, using an image recognition technology on the captured image data.

In a case where it is determined that "the overlap is present", the processor 303 operates the dispersion device to eliminate the overlap of the plurality of drugs 50 in the dose packaging bags 118. By operating the dispersion device, the first camera 308 and the second camera 310 can accurately capture images of the drugs 50 in the dose packaging bags 118. As the dispersion device, publicly known technologies can be applied (for example, Japanese Patent Application Laid-Open No. 2018-029949, etc.). For example, in the publicly known technologies, the conveyance of the dose packaging bags 118 is temporarily stopped in order to capture images of the drugs 50 in the imaging area, and the dispersion device is moved back and forth between upstream and downstream along the conveying direction until an overlap of the drugs 50 is eliminated. The first camera 308 and the second camera 310 capture images of the drugs 50 again. Meanwhile, in a case where it is determined that "the overlap is not present", the dispersion device is not operated, and the first camera 308 and second camera 310 do not capture images of the drugs 50 again.

In determination of the presence or absence of the overlap, for example, the number of the drugs 50 having a specific shape is counted, and it is determined that "the overlap is not present" in a case where the counted number of the drugs 50 matches the number of drugs registered in the prescription data.

Next, with use of the image recognition technology, the processor 303 extracts, from the captured image data, drug information such as the number, shape, size, color, marking, and text of the drugs 50 packaged in the dose packaging bags 118. The processor 303 collates the prescription information from the receipt computer 500 with the extracted drug information, and displays the collation results on the display device 336. The drugs 50 are identified.

On the downstream side of the imaging area, a guide 314 is arranged. The guide 314 guides the dose packaging bags 118 to a conveying path on a lower side.

The drug identification device 300 may include a label printer mechanism 316. A third camera 330 is arranged at a position facing the label printer mechanism 316 across the conveying path. The third camera 330 capture images of the perforations 118C formed in the longitudinal seal part 118A of the dose packaging bag 118 to detect the position of the perforations 118C. Based on the detected position of the perforations 118C, a position at which the label is attached, is adjusted.

The label printer mechanism 316 includes: a supply mechanism 320 which feeds a mount 318 having labels thereon; a label printer 322; a label release mechanism 324; and a winding mechanism 326 which winds the mount. The supply mechanism 320 comprises, for example, a shaft which holds the rolled mount 318 having labels thereon, and a drive motor which rotates the shaft, or the like. For example, the label printer 322 comprises a thermal head printer. The winding mechanism 326 comprises a shaft which winds the mount 318 having no labels thereon, and a drive motor which rotates the shaft, or the like. The mount 318 having labels thereon, is folded such that the tips of the printed labels are released from the mount. The released printed labels pass through a pair of belt conveying mechanisms, and the printed labels are conveyed toward the dose packaging bags 118. The printed labels are attached to the dose packaging bags 118.

Both of the printhead 122 of the packaging mechanism 110 and the label printer mechanism 316 of the drug identification device 300 may be used in combination, or any one of them may be used.

At the downstream side of the label printer mechanism 316, a pair of third conveying rollers 332 is arranged. The third conveying rollers 332 discharge the labeled continuous dose packaging bags 118 from the outlet 334. The dose packaging bags 118 discharged from the outlet 334 are stored in the storing box 400. Although the storing box 400 is shown in the embodiment, a winding device may be arranged instead of the storing box 400. The winding device comprises a winding shaft, and a drive motor which drives the winding shaft, or the like.

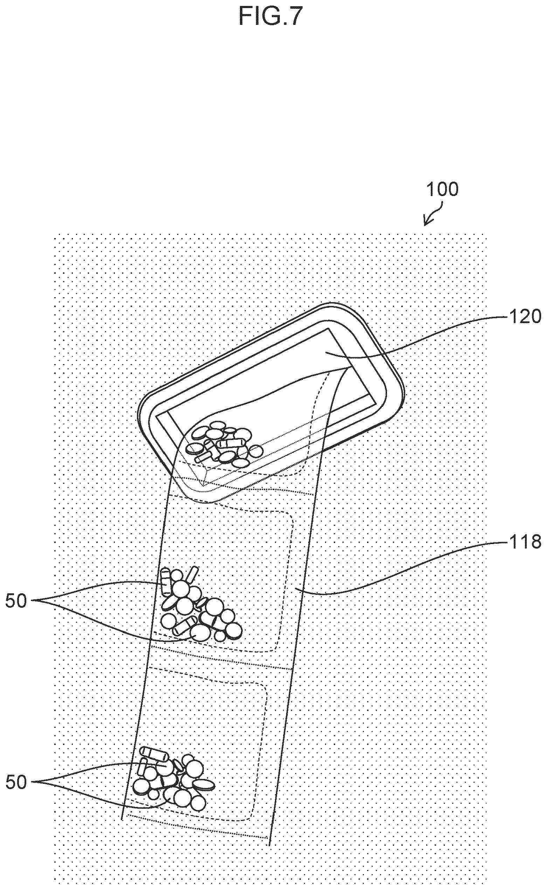

The operation of the drug identification device 300 is described below. Figure 7 shows the dose packaging bags 118 in a state of being discharged from the outlet 120 of the dose packaging device 100. As shown in Figure 7, the outlet 120 is tilted by about 45°. Accordingly, when the dose packaging bags 118 are discharged from the dose packaging device 100, the drugs 50 in the dose packaging bags 118 gather in one corner of the dose packaging bags 118 due to gravity. The plurality of drugs 50 overlap each other.

Thus, when the drugs 50 are just discharged from the dose packaging device 100, the drugs 50 are conveyed to the drug identification device 300 in a state where the drugs gather toward one corner in the dose packaging bags 118. Even in a case where the dispersion device is provided in the imaging area of the drug identification device 300, there is a concern that it takes time to disperse (spread out) the drugs 50. In the case where the dispersion device is operated while the dose packaging bags 118 are stopped, it is important to reduce the time for dispersing the drugs in order to improve throughput of the drug identification device 300.

As shown in Figures 1 and 4, the dose packaging bags 118 discharged from the outlet 120 of the dose packaging device 100, are stored in the storing case 200. A tip of the dose packaging bags 118 is put into the inlet 302 of the drug identification device 300. The cam 222 rotates as the motor 221 is rotated. The coupling member 226 is linearly reciprocated (performs a reciprocating linear motion) by rotation of the cam 222. Since the coupling member 226 is attached to the base plate 214 which can move along the guide rails 212, the base plate 214 moves back and forth in the direction perpendicular to the conveying direction, and the storing case 200 supported by the base plate 214 vibrates in the direction perpendicular to the conveying direction. The rotary motion of the cam 222 is converted into the reciprocating linear motion by the coupling member 226, the base plate 214, and the guide rails 212.

The dose packaging bags 118 are arranged horizontally in the state of being stored in the storing case 200. The storing case 200 is vibrated in the horizontal direction and in the direction perpendicular to the conveying direction. The dose packaging bags 118 in the storing case 200 are vibrated in the horizontal direction and in the direction perpendicular to the conveying direction.

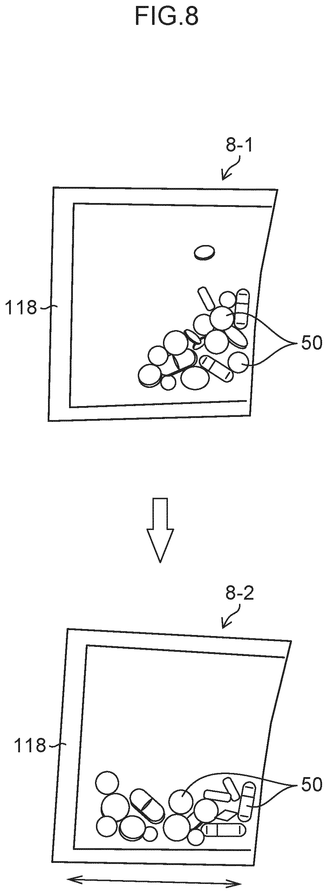

As shown in Part 8-1 in Figure 8, before application of vibration, the drugs 50 are in a state where the drugs 50 gather in one corner of the dose packaging bag 118. Then, as shown in Part 8-2 of Figure 8, the dose packaging bag 118 stored in the storing case 200 is vibrated in the direction perpendicular to the conveying direction so that the drugs 50 can be dispersed in a width direction in the dose packaging bag 118.

Since the storing case 200 stores the continuous dose packaging bags 118, when the storing case 200 is vibrated, all the drugs 50 inside the continuous dose packaging bags 118 can be dispersed at the same time. The dose packaging bags 118 in which the drugs 50 are dispersed in the width direction thereof, are conveyed from the inlet 302 to the cabinet 301.

In the embodiment shown in Figures 1 and 4, an example where the storing case 200 is vibrated after the tip of the dose packaging bags 118 is put into the inlet 302. However, the embodiment is not limited to the example. The storing case 200 may be vibrated before the tip of the dose packaging bags 118 is put into the inlet 302, and then the tip of the dose packaging bags 118 may be put into the inlet 302. The storing case 200 may be vibrated while the dose packaging bags 118 are being fed through the inlet 302, after the dose packaging bags 118 are put into the inlet 302. Until the dose packaging bags 118 stored in the storing case 200 are discharged to the storing box 400, the storing case 200 can be vibrated regularly or irregularly.

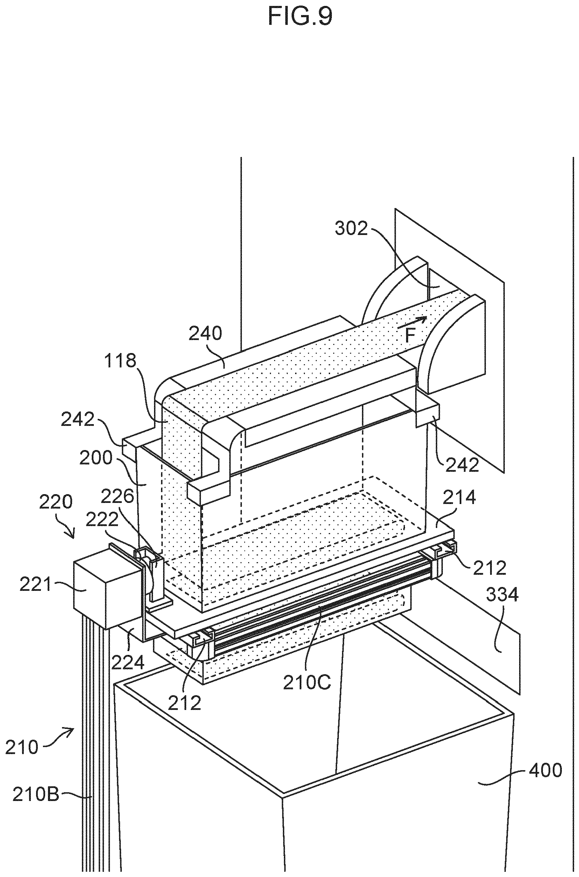

Figure 9 shows a modified example of the storing case 200. As shown in Figure 9, a horizontal guide path 240 is provided above the storing case 200. The horizontal guide path 240 includes legs 242. The legs 242 are mounted on the edge of an opening of the storing case 200. The legs 242 are used to adjust the height such that the horizontal guide path 240 is aligned with the position of the inlet 302. The position of the inlet 302 means a position in a height direction, and the inlet 302 and the horizontal guide path 240 are in the same height position. The same height includes a substantially same height. The horizontal guide path 240 may be positioned in the range of ± 50 mm relative to the inlet 302. In the modified example of the storing case 200, the vibration device 220 can vibrate the dose packaging bags 118 in a state where the dose packaging bags 118 is placed on the horizontal guide path 240. By extending the horizontal conveying path to the inlet 302, the drugs 50 (not shown) in the dose packaging bags 118 are more easily dispersed in the conveying direction.

The processor 506 which implements the receipt computer 500, the processor of the dose packaging machine controller 101, and the processor 303 of the drug identification device 300 in the present embodiment can be implemented by processors shown below. Various types of the processors include: a central processing unit (CPU) that is a generalpurpose processor which functions by executing programs; a programmable logic device (PLD) that is a processor capable of modifying circuit configuration after manufacturing, such as a field programmable gate array (FPGA); and an exclusive electrical circuit that is a processor having a circuit configuration exclusively designed for execution of specific processes, such as an application specific integrated circuit (ASIC). One processor may comprise one of the various processor, or may comprise two or more processors of the same kind or different kinds. For example, one processor may comprise a plurality of FPGAs or a combination of a CPU and an FPGA. A plurality of processors may be implemented by one processor. As an example of a plurality of processors implemented by one processor, firstly, there may be a configuration, as represented by a computer such as a client or a serve, where a combination of one or more CPUs and software constitutes one processor, and the one processor functions as the plurality of processors. Secondary, there may be a configuration using a processor, as represented by a system on chip (SoC) or the like, which implements functions of the entire system including a plurality of processing parts, with a single integrated circuit (IC) chip. In this way, the various processors are implemented using one or more of the various processors in terms of the hardware structure. Furthermore, the hardware structures of the various kinds of processors correspond to an electrical circuit (circuitry) formed by combining circuit elements such as semiconductor elements to be more specific.

In the embodiment, an example where the storing case 200 is vibrated in the horizontal direction and in the direction perpendicular to the conveying direction F has been described. However, the storing case 200 may be vibrated in other directions as long as the drugs 50 in the dose packaging bags 118 can be dispersed. For example, the storing case 200 may be vibrated in the same direction as the conveying direction F, so that the drugs 50 in the dose packaging bags 118 may be dispersed in the longitudinal direction of the dose packaging bags 118.

### Reference Signs List

- 10: Drug audit support system
- 20: Network
- 50: Drug
- 100: Dose packaging device
- 11: Dose packaging machine controller
- 102: Casing
- 104: Feeder
- 106: Hopper
- 108: Charge pipe
- 110: Packaging mechanism
- 112: Dose packaging paper
- 114: Supply mechanism
- 116: Heat seal mechanism
- 116A: Longitudinal heat head
- 116B: Lateral heat head
- 118: Dose packaging bag
- 118A: Longitudinal seal part
- 118B: Lateral seal part
- 118C: Perforations
- 120: Outlet
- 122: Printhead
- 124: Storing case
- 200: Storing case
- 210: Frame
- 210A: Base
- 210B: Post
- 210C: Upper base
- 212: Guide rail
- 214: Baseplate
- 220: Vibration device
- 221: Motor
- 222: Cam
- 224: Bracket
- 226: Coupling member
- 240: Horizontal guide path
- 242: Leg
- 300: Drug identification device
- 301: Casing
- 302: Inlet
- 303: Processor
- 304: First conveying rollers
- 306: Second conveying rollers
- 308: First camera
- 310: Second camera
- 312: Light source
- 314: Guide
- 316: Label printer mechanism
- 318: Mount
- 320: Supply mechanism
- 322: Label printer
- 324: Label release mechanism
- 326: Winding mechanism
- 330: Third camera
- 332: Third conveying rollers
- 334: Outlet
- 336: Display device
- 400: Storing box
- 500: Receipt computer
- 501: Control device
- 502: Display device
- 504: Input device
- 506: Processor
- 508: Storage device
- 510: Communication interface

## Claims

1. A drug identification device comprising:
an inlet configured to receive dose packaging bags in each of which drugs are packaged;
a conveying path configured to convey the dose packaging bags received by the inlet;
an imaging element configured to capture images of the drugs on the conveying path;
a processor configured to identify the drugs based on the images of the drugs captured by the imaging element;
a storing case arranged at an upstream side of the inlet and configured to store the dose packaging bags; and
a vibration device configured to vibrate the storing case.

2. The drug identification device according to claim 1, wherein the vibration device vibrates the storing case in a horizontal direction.

3. The drug identification device according to claim 2, wherein the vibration device vibrates the storing case in a direction perpendicular to a conveying direction of the dose packaging bags.

4. The drug identification device according to any one of claims 1 to 3, wherein the vibration device includes a motor and a cam attached to the motor.

5. The drug identification device according to any one of claims 1 to 4, wherein the storing case includes a horizontal guide path aligned with a position of the inlet.

6. The drug identification device according to any one of claims 1 to 5, further comprising a dispersion device configured to disperse the drugs inside each of the dose packaging bags, on the conveying path.

7. A drug identification method comprising:
a conveying step of conveying on a conveying path, dose packaging bags which are put into an inlet and in each of which drugs are packaged;
an imaging step of capturing images of the drugs on the conveying path; and
an identifying step of identifying the drugs based on the captured images of the drugs; wherein
the drug identification method further comprises a vibrating step of storing the dose packaging bags in a storing case arranged at an upstream side of the inlet, and vibrating the storing case.

8. The drug identification method according to claim 7, wherein in the vibrating step, the storing case is vibrated in a horizontal direction.

9. The drug identification method according to claim 8, wherein in the vibrating step, the storing case is vibrated in a direction perpendicular to a conveying direction of the dose packaging bags.

10. The drug identification method according to any one of claims 7 to 9, wherein in the vibrating step, the storing case is vibrated before the dose packaging bags are put into the inlet.

11. The drug identification method according to any one of claims 7 to 9, wherein in the vibrating step, the storing case is vibrated after the dose packaging bags are put into the inlet.
